# EUROPÄISCHE PATENTSCHRIFT

(11) **EP 2 213 230 B1**
(45) Veröffentlichungstag und Bekanntmachung des Hinweises auf die Patenterteilung: **09.11.2016**
(21) Anmeldenummer: 09001459.8
(22) Anmeldetag: 03.02.2009
(51) Int. Cl.: A61B 5/151

(54) **Stechsystem für ein Stechgerät**
Piercing system for a piercing device
Système de connexion pour un appareil de connexion

(43) Veröffentlichungstag der Anmeldung: 04.08.2010
(73) Patentinhaber: Roche Diabetes Care GmbH, 68305 Mannheim (DE); F.Hoffmann-La Roche AG, 4070 Basel (CH)
(72) Erfinder: Konya, Ahmet, 67165 Waldsee (DE)
(74) Vertreter: Twelmeier Mommer & Partner

(56) Entgegenhaltungen:
- EP-A- 1 690 496
- EP-A- 1 881 322
- EP-A- 1 992 284
- WO-A-2006/059241
- WO-A-2007/077212
- US-A1- 2007 038 150

## Beschreibung

Die Erfindung geht aus von einem Stechsystem mit den im Oberbegriff des Anspruchs 1 angegebenen Merkmalen. Ein Stechsystem mit den im Oberbegriff des Anspruchs 1 angegebenen Merkmalen ist aus der WO 2007/077212 A2 bekannt.

Aus der WO 2005/107596 A2 ist ein Stechsystem bekannt, bei dem auf einem Trägerband zwischen Stechelementen jeweils ein Testelement angeordnet ist. Auf das Trägerband sind einzelne Folienstreifen aufgeklebt, die jeweils ein Stechelement bedecken und Fenster aufweisen. Vor Gebrauch muss ein Stechelement auf dem Trägerband verschoben werden, damit seine Spitze nicht mehr von einem Folienstreifen bedeckt ist. Dies erfordert ein Stechgerät mit einer aufwändigen Mechanik. Zudem müssen die Folienstreifen mit ihren Fenstern mit einer hohen Präzision auf den einzelnen Stechelementen platziert werden, was einen erheblichen Fertigungsaufwand verursacht.

Bekannt sind auch Stechsysteme - beispielsweise aus der EP 1 360 935 B1 - bei denen Testelemente integral mit Stechelementen ausgebildet sind. Nachteilig an derartigen Systemen ist jedoch, dass durch empfindliche Nachweißreagenzien des Testelements die Sterilisation des Stechelements erschwert wird. Auch bei derartigen System erfordert das Entfernen der Sterilverpackung eine aufwändige Mechanik, da eventuell verbleibende Reste der Sterilverpackung die Probenaufnahme behindern können, indem ein Kapillarkanal teilweise verdeckt wird oder durch Folienreste störende Kapillareffekte auftreten können.

Aufgabe der Erfindung ist es, einen Weg aufzuzeigen, wie ein Stechsystem kostengünstig verwirklicht werden kann.

Diese Aufgabe wird durch ein Stechsystem mit den im Anspruch 1 angegebenen Merkmalen gelöst. Vorteilhafte Weiterbildungen der Erfindung sind Gegenstand von Unteransprüchen.

Erfindungsgemäß werden zwei verschiedene Trägerbänder verwendet, nämlich ein Stechelemente-Trägerband, das die Stechelemente trägt, und ein Testelemente-Trägerband, das die Testelemente trägt. Die beiden Trägerbänder liegen mit Bandabschnitten, die unbenutzte Testelemente beziehungsweise unbenutzte Stechelemente tragen, aufeinander. Diese Maßnahme ermöglicht eine kostengünstige Fertigung, da Stechelemente-Trägerbänder mit Stechelementen und Testelemente-Trägerbänder mit Testelementen separat gefertigt werden können. Beispielsweise können bei der Herstellung ein Stechelemente-Trägerband mit Stechelementen und ein Testelemente-Trägerband mit Testelementen aufeinander gelegt und anschließend zickzackförmig zu einem Stapel gefaltet oder zu einer Rolle aufgewickelt werden.

Bevorzugt bedeckt das Testelemente-Trägerband die unbenutzten Stechelemente. Besonderes bevorzugt bildet das Testelemente-Trägerband dabei eine Sterilverpackung für die unbenutzten Stechelemente.

Eine weitere vorteilhafte Weiterbildung der Erfindung sieht vor, dass die beiden Trägerbänder mit Bandabschnitten aufeinander liegen, zwischen denen sich unbenutzte Testelemente und unbenutzte Stechelemente befinden. Auf diese Weise können sowohl die Stechelemente als auch die Testelemente durch die beiden Trägerbänder vor schädlichen Umwelteinflüssen geschützt und durch Trennen der beiden Trägerbänder freigelegt werden. Möglich ist es beispielsweise aber auch, die Testelemente und die Stechelemente jeweils in gleicher Orientierung auf den beiden Trägerbändern anzuordnen, so dass beispielsweise nur die Stechelemente bedeckt werden, indem man das Testelemente-Trägerband auf das Stechelemente-Trägerbandes legt.

Eine weitere vorteilhafte Weiterbildung eines erfindungsgemäßen Stechsystems sieht eine Bandführung vor, welche das Testelemente-Trägerband von dem Stechelemente-Trägerband trennt und die beiden Trägerbänder auf unterschiedlichen Wegen führt. Bevorzugt enthalten die unterschiedlichen Wege jeweils mindestens einen gekrümmten Abschnitt, auf dem die beiden Trägerbänder in einem Abstand von einander geführt werden. Erfindungsgemäß kann ein Versatz zwischen den Stechelementen und den Testelementen ausgeglichen, so dass ein Testelement für eine Probenaufnahme zu einem Stechelement gelangen kann.

Zu einem erfindungsgemäßen Stechsystem gehören bevorzugt ein Handgerät und eine Bandkassette, die in das Stechgerät einsetzbar ist. Eine solche Bandkassette enthält ein Stechelemente-Trägerband, das mehrere Stechelemente trägt und ein Testelemente-Trägerband, das mehrere Testelemente trägt, wobei die beiden Trägerbänder aufeinander liegen, insbesondere übereinander aufgewickelt oder als zickzackförmig gefalteter Stapel. Es ist aber auch möglich, ein erfindungsgemäßes Stechsystem als ein Stechgerät auszuführen, bei dem ein Bandaustausch nicht vorgesehen ist. Ein solches Handgerät wird als Wegwerfgerät entsorgt, sobald der darin enthaltene Vorrat an Stechelementen und Testelementen aufgebraucht ist.

Weitere Einzelheiten und Vorteile der Erfindung werden an einem Ausführungsbeispiel unter Bezugnahme auf die beigefügte Zeichnung erläutert. Es zeigen:
- Fig. 1: eine schematische Darstellung eines erfindungsgemäßen Stechsystems; und
- Fig. 2: eine schematische Darstellung eines Stechelemente-Trägerbandes mit unbenutzten Stechelementen und eines an ihm klebenden Testelemente-Trägerbandes mit unbenutzten Testelementen.

Zu dem dargestellten Stechsystem gehören ein Stechelemente-Trägerband 1, das Stechelemente 2 trägt, und ein Testelemente-Trägerband 3, das Testelemente 4 mit Nachweisreagenzien zur Untersuchung einer durch einen Stich gewonnenen Körperflüssigkeitsprobe trägt. Die beiden Trägerbänder 1, 3 sind bei dem dargestellten Ausführungsbeispiel übereinander in einer Vorratskammer auf einer Rolle 5 aufgewickelt, könnten jedoch beispielsweise auch zu einem Stapel gefaltet sein.

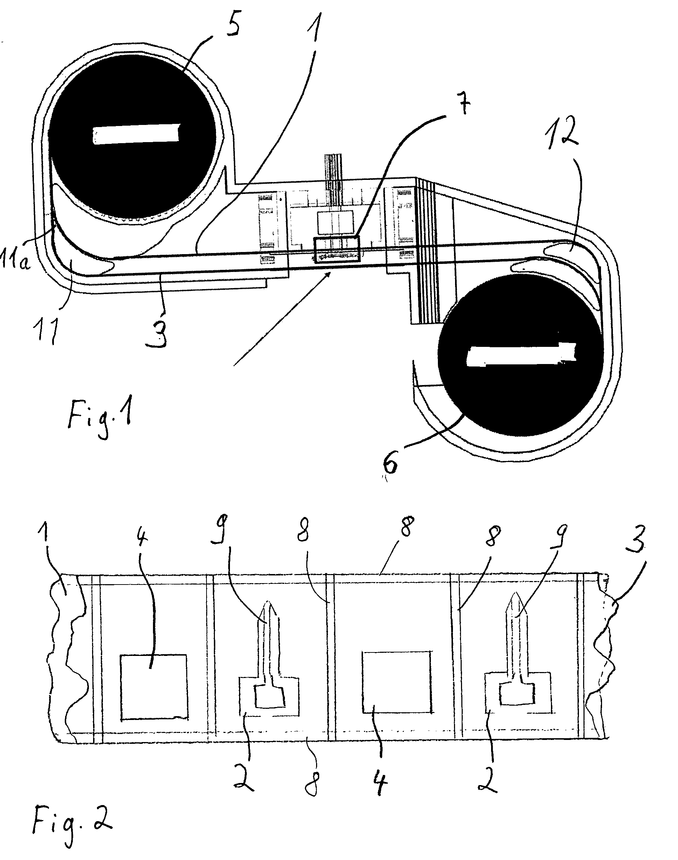

Mit einer Bandtransporteinrichtung, die bei dem dargestellten Ausführungsbeispiel eine Wickeleinrichtung 6 ist, können die beiden Trägerbänder 1, 3 transportiert und die Stechelemente 2 nacheinander in eine Gebrauchsposition gebracht werden, in der sie von einem Stechantrieb 7 in eine Stechbewegung versetzt werden können. Bei dem dargestellten Ausführungsbeispiel erfolgt die Stechbewegung senkrecht zur Zeichenebene der Figur 1.

Das dargestellte Stechsystem hat eine Bandführung, welche das Testelemente-Trägerband 3 von dem Stechelemente-Trägerband trennt 1 und die beiden Trägerbänder 1, 3 auf unterschiedlichen Wegen in einem Abstand voneinander führt. Eine solche Bandführung mit einer Trennung der beiden Trägerbänder 1, 3 wird bei dem dargestellten Ausführungsbeispiel mit einem Bandführungselement 11 erreicht, das zwischen dem Testelemente-Trägerband 3 und dem Stechelemente-Trägerband 1 angeordnet ist. Das Bandführungselement 11 kann beispielsweise eine keilförmige Frontseite 11a haben, auf welche die noch verbunden Trägerbänder 1, 3 beim Bandtransport zu bewegt werden.

Um die beiden Trägerbänder 1, 3 nach der Trennung in einem Abstand voneinander zu führen, ist das Bandführungselement 11 gekrümmt ausgebildet. Die beiden Trägerbänder 1, 3 werden auf unterschiedlichen Wegen geführt. Die unterschiedlichen Wege des Testelemente-Trägerbands 3 und des Stechelemente-Trägerbands 1 umfassen dabei jeweils mindestens einen gekrümmten Wegabschnitt, wobei der gekrümmte Wegabschnitt des Testelemente-Trägerbands 3 und der gekrümmte Wegabschnitt des Stechelemente-Trägerbands 1 nebeneinander verlaufen und die beiden gekrümmten Wegabschnitte jeweils in der derselben Richtung gekrümmt sind.

Wie Figur 1 zeigt, werden die Trägerbänder 1, 3 also gebogen und entlang einer Kurve geführt. Hinter der Gebrauchsposition der Stechelemente 2 werden die beiden Trägerbänder 1, 3 wieder zusammengeführt und übereinander auf der Wickeleinrichtung 6 aufgewickelt. Zum Zusammenführen der beiden Trägerbänder 1, 3 ist ein weiteres Bandführungselement 12 vorgesehen, das ebenfalls zwischen den beiden Trägerbändern 1, 3 angeordnet ist. Das Bandführungselement 12 ist in umgekehrter Richtung wie das Bandführungselement 11 gekrümmt.

Der Abstand, in dem die beiden Trägerbänder 1, 3 zwischen den beiden Bandführungselementen 11, 12 geführt werden, ist bevorzugt so groß, dass durch einen zwischen den beiden Trägerbändern 1, 3 gebildeten Spalt keine Kapillarkräfte auf eine von einem Testelement 4 aufgenommene Körperflüssigkeitsprobe ausgeübt werden. Beispielsweise können die beiden Trägerbänder 1, 3 parallel zueinander in einem Abstand von mindestens 2 mm, insbesondere mindestens 5 mm geführt werden.

Figur 2 zeigt aufeinander liegende Bandabschnitte der beiden Trägerbänder 1, 3 mit unbenutzten Testelementen 4 und unbenutzten Stechelementen 4. In Bandrichtung gesehen befinden sich die Testelemente 4 jeweils zwischen den Stechelementen 2, die beiden Trägerbänder 1, 3 liegen also versetzt aufeinander. Der Abstand zwischen den unbenutzten Stechelementen 2 und den unbenutzten Testelementen stimmt bei dem dargestellten Ausführungsbeispiel mit dem Abstand überein, in dem die beiden Trägerbänder 1, 3 nach der Trennung, also beispielsweise zwischen den Bandführungselementen 11, 12 geführt werden.

Das Testelemente-Trägerband 3 bedeckt die unbenutzten Stechelemente 2. Die beiden Trägerbänder 1 ,3 kleben mit Bandabschnitten, zwischen denen sich die unbenutzten Testelemente 4 und die unbenutzten Stechelemente 2 befinden, aufeinander. Klebeflächen 8, welche die Stechelemente 2 und die Testelemente 4 umgeben, sind in Figur 2 eingezeichnet.

Die unbenutzten Stechelemente 2 sind auf diese Weise in Kammern abgeordnet, deren Oberseiten von dem Testelemente-Trägerband 3 und deren Unterseiten von dem Stechelemente-Trägerband 1 gebildet sind. Die Stechelemente 2 können so mit geringem Aufwand steril verpackt werden. Ein Öffnen der Kammern geschieht durch die Trennung der beiden Trägerbänder 1, 3 automatisch, so dass die Stechelemente 2 bei Erreichen ihrer Gebrauchsposition betriebsbereit sind. Bevorzugt sind die beiden Trägerbänder 1, 3 ähnlich wie Klebeetiketten auf Releasepapier lösbar miteinander verklebt, so dass sich die Trägerbänder 1, 3 leicht voneinander trennen lassen. Die Stechelemente 2 und die Testelemente 4 umschließenden Klebeflächen 8 der Trägerbänder 1, 3 sind in Figur 2 dargestellt.

Bei dem dargestellten Ausführungsbeispiel bedeckt das Stechelemente-Trägerband 1 die unbenutzten Testelemente 4. Auf diese Weise können die Testelemente 4 ohne zusätzlichen Aufwand vor schädlichen Umwelteinflüssen geschützt werden. Die Testelemente 4 sind ähnlich wie die Stechelemente 2 in Kammern angeordnet, welche durch die aufeinander klebenden Trägerbänder 1, 3 gebildet sind.

Durch die in Figur 1 erfindungsgemäße Bandführung, welche die beiden Trägerbänder auf unterschiedlichen Wegen führt, kann ein Testelement 4 zu einem Stechelement 2 gebracht werde, um eine Körperflüssigkeitsprobe aufzunehmen. Die Testelemente 4 und die Stechelemente 2 legen nämlich von der Stelle, an der die beiden Trägerbänder 1, 3 von einander getrennt werden, bis zur Übergabe einer Probe von einem Stechelement 2 auf ein Testelement 4, unterschiedlich lange Wegstrecken zurück. Die Wegstrecken unterscheiden sich dabei um den in Bandrichtung gemessenen Abstand zwischen Testelementen 4 und Stechelementen 2 bevor die beiden Trägerbänder 1, 3 getrennt werden. Durch die unterschiedlich langen Wegstrecken wird also ein Versatz zwischen dem Stechelemente-Trägerband 1 und dem Testelemente-Trägerband 3 ausgeglichen.

Bei dem dargestellten Ausführungsbeispiel werden die unterschiedlich langen Wegstrecken dadurch bewirkt, dass die beiden Trägerbänder 1, 3 nach der Trennung entlang einer Kurve geführt werden. Auf diese Weise ergibt sich für das in Krümmungsrichtung der Kurve innen geführte Trägerband, in Figur 1 also das Trägerband 1, eine kürzere Wegstrecke als für das in Krümmungsrichtung außen geführte Trägerband, in Figur 1 also das Trägerband 3.

Welches der beiden Trägerbänder 1, 3 innen geführt wird und welches außen geführt wird, spielt an sich keine Rolle. Durch die unterschiedlichen Wege lässt sich stets erreichen, dass ein Testelement 4 zur Probenaufnahme zu einem Stechelement 2 geführt wird. Bei dem dargestellten Ausführungsbeispiel wird durch die unterschiedlichen Wege ein Testelement 4 über einem Stechelement 2 positioniert, das sich in der Gebrauchsposition, beispielsweise in einer Aufnahme des Stechantriebs 7, befindet. Auf diese Weise kann ein Stechelement 2 bei einem Stich mit seiner Probenaufnahmeeinrichtung 9, beispielsweise einem Kapillarkanal, eine Körperflüssigkeitsprobe aufnehmen und anschließend auf ein Testelement 4 übertragen. Bevorzugt werden bei einem Stich nur das Stechelement 2 und das Stechelemente-Trägerband 1, aber nicht das Testelemente-Trägerband 3. Bevorzugt wird zur Probenübergabe eine Bewegung des Stechelements oder des Testelements senkrecht zur Stichrichtung ausgeführt. Beispielsweise kann der Stechantrieb 7 das Stechelemente-Trägerband 1 nach oder während einem Stich gegen das Testelemente-Trägerband 3 drücken. Bevorzugt bleibt das Stechelement 2 bei einem Stich mit dem Stechelemente-Trägerband 1 verbunden. Nach einer Probenaufnahme kann das Testelement gegen eine Messeinrichtung, vorzugsweise eine optische Messeinrichtung, drücken. Die Messeinrichtung kann beispielsweise ein CMOS Sensor sein.

Bei dem dargestellten Ausführungsbeispiel nimmt ein Testelement 4 eine Körperflüssigkeitsprobe von einem Stechelement 2 auf, das sich in der Gebrauchsposition befindet. Prinzipiell ist es aber auch möglich, eine Probenübergabe an einer anderen Stelle durchzuführen, also nach einem Stich die beiden Trägerbänder 1, 3 noch etwas weiterzutransportieren, bevor ein Testelement 4 in Kontakt mit der Probenaufnahmeeinrichtung 9 eines Stechelements 2 gebracht wird. Beispielsweise können zusätzliche Bandführungselemente vorgesehen sein, welche gemeinsam einen Spalt bilden, durch den die beiden Trägerbänder 1, 3 geführt werden, so dass ein Stechelement 2 und ein Testelement 4 in Kontakt kommen.

Hinter der Gebrauchsposition werden das Stechelemente-Trägerband 1 und das Testelemente-Trägerband 3 wieder zusammengeführt. Dabei werden die Trägerbänder 1, 3 bevorzugt so geführt, dass die zuvor unterschiedlich langen Wegstrecken wieder ausgeglichen werden, also die ursprüngliche Anordnung der Testelemente 4 zwischen den Stechelementen 2 wieder hergestellt wird. Bei dem dargestellten Ausführungsbeispiel wird dies dadurch erreicht, dass die beiden Trägerbänder 1, 3 hinter der Gebrauchsposition in einem Abstand entlang einer Kurve geführt werden, die umgekehrt zu der vorausgehenden Kurve gekrümmt ist, also das Bandführungselement 12 umgekehrt wie das Bandführungselement 11 gekrümmt ist. Auf diese Weise können die Trägerbänder 1, 3 mit Bandabschnitten, die benutzte Testelemente 4 beziehungsweise Stechelemente 2 tragen, auf der Wickeleinrichtung 6 problemlos aufeinander gewickelt werden. Bei dem dargestellten Ausführungsbeispiel wickelt die Wickeleinrichtung 6 die Trägerbänder 1, 3 also in einer Wickelrichtung auf, welche der Wickelrichtung entgegengesetzt ist, in der die Trägerbänder 1, 3 mit Bandabschnitten, die unbenutzte Testelemente 4 beziehungsweise unbenutzte Stechelemente 2 tragen, aufgewickelt sind. Möglich ist es aber auch, die Rolle 5 und die Wickeleinrichtung 6 wie bei herkömmlichen Tonbandkassetten spiegelsymmetrisch zueinander anzuordnen.

### Bezugszahlen

- 1: Stechelemente-Trägerband
- 2: Stechelemente
- 3: Testelemente-Trägerband
- 4: Testelemente
- 5: Rolle
- 6: Wickeleinrichtung
- 7: Stechantrieb
- 8: Klebefläche
- 9: Probenaufnahmeeinrichtung
- 10 11: Bandführungselement
- 11 a: Frontseite
- 12: Bandführungselement

## Patentansprüche

1. Stechsystem mit
einem Stechelemente-Trägerband (1), das mehrere Stechelemente (2) trägt, einer Bandtransporteinrichtung (6), um durch Transport des Stechelemente-Trägerbandes (1) die Stechelemente (2) nacheinander in eine Gebrauchsposition zu bringen,
einem Stechantrieb (7), um ein Stechelement (2), das sich in der Gebrauchsposition befindet, in eine Stechbewegung zu versetzten,
Testelementen (4), bevorzugt mit Nachweisreagenzien, zur Untersuchung einer durch einen Stich gewonnenen Körperflüssigkeitsprobe,
einem Testelemente-Trägerband (3), das die Testelemente (4) trägt, wobei die beiden Trägerbänder (1, 3) mit Bandabschnitten, die unbenutzte Testelemente (4) bzw. unbenutzte Stechelemente (2) tragen, aufeinander liegen,
einer Bandführung, welche das Testelemente-Trägerband (3) von dem Stechelemente-Trägerband (1) trennt und die beiden Trägerbänder (1, 3) auf unterschiedlichen Wegen führt, wobei durch die unterschiedlichen Wege ein Testelement (4) zur Probenaufnahme zu einem Stechelement (2) geführt wird, **dadurch gekennzeichnet, dass** sich die Testelemente (4) vor der Trennung des Testelemente-Trägerbands (3) von dem Stechelemente-Trägerband (1) in Bandrichtung gesehen jeweils zwischen den Stechelementen (2) befinden, und dass Testelemente (4) und Stechelemente (2) von der Stelle, an welcher die Trägerbänder (1, 3) voneinander getrennt werden, bis zur Übergabe einer Probe von einem Stechelement (2) auf ein Testelement (4), unterschiedlich lange Wegstrecken zurücklegen.

2. Stechsystem nach Anspruch 1, **dadurch gekennzeichnet, dass** das Testelemente-Trägerband (3) die unbenutzten Stechelemente (2) bedeckt.

3. Stechsystem nach einem der vorstehenden Ansprüche, **dadurch gekennzeichnet, dass** die unbenutzten Stechelemente (2) in Kammern angeordnet sind, die zwischen dem Testelemente-Trägerband (3) und dem Stechelemente-Trägerband (1) gebildet sind.

4. Stechsystem nach einem der vorstehenden Ansprüche, **dadurch gekennzeichnet, dass** das Stechelemente-Trägerband (1) die unbenutzten Testelemente (4) bedeckt.

5. Stechsystem nach einem der vorstehenden Ansprüche, **dadurch gekennzeichnet, dass** die beiden Trägerbänder (1, 3) mit Bandabschnitten, die unbenutzte Testelemente (4) bzw. unbenutzte Stechelemente (2) tragen, aufeinander kleben.

6. Stechsystem nach einem der vorstehenden Ansprüche **dadurch gekennzeichnet, dass** die Stechelemente (2) eine Probenaufnahmeeinrichtung (9) aufweisen.

7. Stechsystem nach Anspruch 6, **dadurch gekennzeichnet, dass** zur Übergabe einer Probe von einem Stechelement (2) zu einem Testelement (4) das Stechelement (2) und das Testelement senkrecht zur Stichrichtung aufeinander zu bewegt werden.

8. Stechsystem nach einem der vorstehenden Ansprüche, **dadurch gekennzeichnet, dass** die unterschiedlichen Wege des Testelemente-Trägerbands (3) und des Stechelemente-Trägerbands (1) jeweils mindestens einen gekrümmten Wegabschnitt umfassen, wobei der gekrümmte Wegabschnitt des Testelemente-Trägerbands (3) und der gekrümmte Wegabschnitt des Stechelemente-Trägerbands (1) in einem Abstand von einander nebeneinander verlaufen und in der derselben Richtung gekrümmt sind.

9. Stechsystem nach einem der vorstehenden Ansprüche, **dadurch gekennzeichnet, dass** zwischen dem Stechelemente-Trägerband (1) und dem Testelemente-Trägerband (2) ein Bandführungselement (11) angeordnet ist.

10. Stechsystem nach einem vorstehenden Ansprüche, **dadurch gekennzeichnet, dass** die Bandführung das Stechelemente-Trägerband (1) hinter der Gebrauchsposition der Stechelemente (2) in entgegen gesetzter Richtung wie vor der Gebrauchsposition biegt.

## Claims

1. A lancing system, comprising
a lancing elements carrier tape (1), which carries a plurality of lancing elements (2),
a tape transport unit (6) for moving the lancing elements (2) consecutively into a usage position by transporting the lancing elements carrier tape (1),
a lancing drive (7) for causing a lancing element (2) located in the usage position to perform a lancing movement,
test elements (4), preferably with detection reagents, for analyzing a body fluid sample obtained by a puncture,
a test elements carrier tape (3), which carries the test elements (4), wherein the two carrier tapes (1, 3) are located on top of one another with tape sections that carry unused test elements (4) or unused lancing elements (2),
a tape guide, which separates the test elements carrier tape (3) from the lancing elements carrier tape (1) and guides the two carrier tapes (1, 3) on different paths, whereby the different paths lead a test element (4) to a lancing element (2) in order to receive a sample,
**characterized in that** the test elements (4) are located between the lancing elements (2), respectively, as viewed in the tape direction, prior to separating the test elements carrier tape (3) from the lancing elements carrier tape (1), and **in that** test elements (4) and lancing elements (2) travel distances having differing lengths from the point at which the carrier tapes (1, 3) are separated from one another until a sample is transferred from a lancing element (2) onto a test element (4).

2. The lancing system according to claim 1, **characterized in that** the test elements carrier tape (3) covers the unused lancing elements (2).

3. A lancing system according to any one of the preceding claims, **characterized in that** the unused lancing elements (2) are disposed in chambers formed between the test elements carrier tape (3) and the lancing elements carrier tape (1).

4. A lancing system according to any one of the preceding claims, **characterized in that** the lancing elements carrier tape (1) covers the unused test elements (4).

5. A lancing system according to any one of the preceding claims, **characterized in that** the two carrier tapes (1, 3) adhere to one another with tape sections that carry unused test elements (4) or unused lancing elements (2).

6. A lancing system according to any one of the preceding claims, **characterized in that** the lancing elements (2) comprise a sample receiving unit (9).

7. The lancing system according to claim 6, **characterized in that** the lancing element (2) and the test element are moved toward one another perpendicular to the puncture direction to transfer a sample from a lancing element (2) to a test element (4).

8. The lancing system according to any one of the preceding claims, **characterized in that** the different paths of the test elements carrier tape (3) and of the lancing elements carrier tape (1) each comprise at least one curved path section, wherein the curved path section of the test elements carrier tape (3) and the curved path section of the lancing elements carrier tape (1) run side by side spaced from one another and are curved in the same direction.

9. A lancing system according to any one of the preceding claims, **characterized in that** a tape guide element (11) is disposed between the lancing elements carrier tape (1) and the test elements carrier tape (2).

10. A lancing element according to any one of the preceding claims, **characterized in that** behind the usage position of the lancing elements (2) the tape guide bends the lancing elements carrier tape (1) in the opposite direction as before the usage position.

## Revendications

1. Système de piqûre avec
une bande porteuse d'éléments de piqûre (1), qui porte plusieurs éléments de piqûre (2),
un dispositif de transport de bande (6), pour amener les éléments de piqûre (2) successivement dans une position d'utilisation grâce à un transport de la bande porteuse d'éléments de piqûre (1),
un entraînement de piqûre (7), pour déplacer dans un mouvement de piqûre, un élément de piqûre (2) qui se trouve dans la position d'utilisation,
des éléments de test (4), comprenant de manière préférée des réactifs de mise en évidence, destinés à examiner un échantillon de fluide corporel obtenu grâce à une piqûre,
une bande porteuse d'éléments de test (3) qui porte les éléments de test (4), dans lequel les deux bandes porteuses (1, 3), comprenant des sections de bande qui portent des éléments de test (4) inutilisés ou bien des éléments de piqûre (2) inutilisés, reposent l'une sur l'autre,
un guidage de bande, qui sépare la bande porteuse d'éléments de test (3) de la bande porteuse d'éléments de piqûre (1) et qui guide les deux bandes porteuses (1, 3) sur des voies différentes, dans lequel un élément de test (4) destiné à un prélèvement d'échantillon est guidé vers un élément de piqûre (2) grâce aux voies différentes,
**caractérisé en ce que** les éléments de test (4) se trouvent respectivement entre les éléments de piqûre (2), vus dans le sens de la bande, avant la séparation de la bande porteuse d'éléments de test (3) par rapport à la bande porteuse d'éléments de piqûre (1), et
des éléments de test (4) et des éléments de piqûre (2) parcourent des distances de longueurs différentes à partir du point au niveau duquel les bandes porteuses (1, 3) sont séparées l'une de l'autre jusqu'au transfert d'un échantillon depuis un élément de piqûre (2) jusque sur un élément de test (4).

2. Système de piqûre selon la revendication 1, **caractérisé en ce que** la bande porteuse d'éléments de test (3) recouvre les éléments de piqûre (2) inutilisés.

3. Système de piqûre selon l'une quelconque des revendications précédentes, **caractérisé en ce que** les éléments de piqûre inutilisés (2) sont agencés dans des chambres qui sont formées entre la bande porteuse d'éléments de test (3) et la bande porteuse d'éléments de piqûre (1).

4. Système de piqûre selon l'une quelconque des revendications précédentes, **caractérisé en ce que** la bande porteuse d'éléments de piqûre (1) recouvre les éléments de test (4) inutilisés.

5. Système de piqûre selon l'une quelconque des revendications précédentes, **caractérisé en ce que** les deux bandes porteuses (1, 3) comprenant des sections de bande qui portent des éléments de test (4) inutilisés ou bien des éléments de piqûre (2) inutilisés se collent l'une sur l'autre.

6. Système de piqûre selon l'une quelconque des revendications précédentes, **caractérisé en ce que** les éléments de piqûre (2) présentent un dispositif d'accueil d'échantillon (9).

7. Système de piqûre selon la revendication 6, **caractérisé en ce que** l'élément de piqûre (2) et l'élément de test sont déplacés l'un sur l'autre perpendiculairement au sens de piqûre en vue d'un transfert d'un échantillon depuis un élément de piqûre (2) jusqu'à un élément de test (4).

8. Système de piqûre selon l'une quelconque des revendications précédentes, **caractérisé en ce que** les trajets différents de la bande porteuse d'éléments de test (3) et de la bande porteuse d'éléments de piqûre (1) comprennent respectivement au moins une section de trajet courbe, dans lequel la section de trajet courbe de la bande porteuse d'éléments de test (3) et la section de trajet courbe de la bande porteuse d'éléments de piqûre (1) sont juxtaposées en présentant un écart l'une par rapport à l'autre et sont courbées dans la même direction.

9. Système de piqûre selon l'une quelconque des revendications précédentes, **caractérisé en ce qu'**un élément de guidage de bande (11) est agencé entre la bande porteuse d'éléments de piqûre (1) et la bande porteuse d'éléments de test (2).

10. Système de piqûre selon l'une quelconque des revendications précédentes, **caractérisé en ce que** le guidage de bande recourbe la bande porteuse d'éléments de piqûre (1) après la position d'utilisation des éléments de piqûre (2) dans le sens inverse de celui dans lequel elle est courbée avant la position d'utilisation.
